(19) Europäisches Patentamt
European Patent Office
Office européen des brevets

(11) Veröffentlichungsnummer: **0 126 249**
**A1**

(12) # EUROPÄISCHE PATENTANMELDUNG

(21) Anmeldenummer: **84103626.2**

(22) Anmeldetag: **02.04.84**

(51) Int. Cl.³: **C 07 F 9/40**, C 07 F 9/32, C 07 F 9/65, A 01 N 57/18

(30) Priorität: **12.04.83 DE 3313070**

(43) Veröffentlichungstag der Anmeldung: **28.11.84 Patentblatt 84/48**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Pfister, Theodor, Dr., Lichtenbergerstrasse 30, D-4019 Monheim (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch Gladbach 2 (DE)**
Erfinder: **Hänssler, Gerd, Dr., Heymannstrasse 40, D-5090 Leverkusen 1 (DE)**

(54) **Chlorierte Phosphorylmethylcarbonyl-Derivate, Verfahren zu ihrer Herstellung und ihre Verwendung in Pflanzenschutzmitteln.**

(57) Die Erfindung betrifft neue Phosphorylmethylcarbonyl-Derivate der allgemeinen Formel

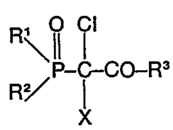

(I)

in welcher $R^1$ für gegebenenfalls substituierte Reste aus der Reihe Alkyl, Aryl, Aralkyl, Alkoxy und Aralkoxy steht, $R^2$ für gegebenenfalls substituierte Reste aus der Reihe Alkoxy oder Aralkoxy steht, oder $R^1$ und $R^2$ gemeinsam für einen Alkandioxy-Rest stehen, X für Wasserstoff oder Chlor steht, $R^3$ für die Gruppierung

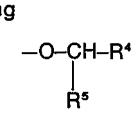

steht, in welcher $R^4$ für Wasserstoff oder für gegebenenfalls substituiertes Alkyl steht und $R^5$ für einen gegebenenfalls substituierten Alkoxycarbonyl-Rest oder für einen gegebenenfalls substituierten Pyrazolyl-Rest steht; oder $R^3$ für den Fall, daß X für Chlor steht, auch für gegebenenfalls substituierte Reste aus der Reihe Alkyl, Cycloalkyl, Alkylamino oder Cycloalkylamino steht, verschiedene Verfahren zu ihrer Herstellung und ihre Verwendung als Pflanzenschutzmittel, insbesondere als Herbizide und Fungizide.

EP 0 126 249 A1

ACTORUM AG

BAYER AKTIENGESELLSCHAFT 5090 Leverkusen, Bayerwerk
Konzernverwaltung RP
Patentabteilung Bi/Mt
Ib

# Chlorierte Phosphorylmethylcarbonyl-Derivate, Verfahren zu ihrer Herstellung und ihre Verwendung in Pflanzenschutzmitteln

Die Erfindung betrifft neue Phosphorylmethylcarbonyl-Derivate, mehrere Verfahren zu ihrer Herstellung und ihre Verwendung als Pflanzenschutzmittel, insbesondere als Herbizide und Fungizide.

Es ist bekannt, daß bestimmte chlorierte Phosphorylessigsäureester als Herbizide verwendet werden können (vgl. z.B. EP 1018). So kann z.B. 2,2-Dichlor-2-(diethoxyphosphoryl)-essigsäure-(1-methyl)-ethylester zur Unkrautbekämpfung eingesetzt werden. Außerdem ist bekannt, daß

bestimmte Bis-dithiocarbamate, wie z.B. Zinkethylen-bis-dithiocarbamat, fungizid wirksam sind (vgl. US-PS 24 57 674 und 30 50 439). Die Wirkung dieser Verbindungen ist jedoch insbesondere bei niedrigen Aufwandmengen und Konzentrationen nicht immer ausreichend.

Es wurden nun neue chlorierte Phosphorylmethylcarbonyl-Derivate der Formel (I) gefunden,

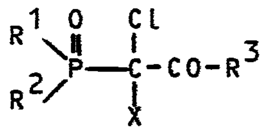

(I)

in welcher

$R^1$ für gegebenenfalls substituierte Reste aus der Reihe Alkyl, Aryl, Aralkyl, Alkoxy und Aralkoxy steht,

$R^2$ für gegebenenfalls substituierte Reste aus der Reihe Alkoxy oder Aralkoxy steht, oder

$R^1$ und $R^2$ gemeinsam für einen Alkandioxy-Rest stehen,

X für Wasserstoff oder Chlor steht,

$R^3$ für die Gruppierung 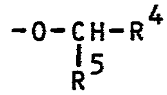 steht,

in welcher

$R^4$ für Wasserstoff oder für gegebenenfalls substituiertes Alkyl steht und

$R^5$ für einen gegebenenfalls substituierten Alkoxycarbonyl-Rest oder für einen gegebenenfalls substituierten Pyrazolyl-Rest steht; oder

$R^3$ für den Fall, daß X für Chlor steht, auch für gegebenenfalls substituierte Reste aus der Reihe Alkyl, Cycloalkyl, Alkylamino oder Cycloalkylamino steht.

Man erhält die neuen Verbindungen der Formel (I), indem man

a) Dichlormethanphosphonsäure-Derivate der Formel (II),

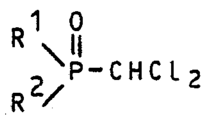 (II)

in welcher

$R^1$ und $R^2$ für gegebenenfalls substituierte Reste aus der Reihe Alkoxy oder Aralkoxy stehen, oder

$R^1$ und $R^2$ gemeinsam für einen Alkandioxy-Rest stehen,

mit Säurehalogeniden der Formel (III),

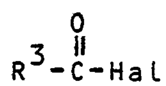 (III)

in welcher

$R^3$ die oben angegebene Bedeutung hat und

Hal für Chlor oder Brom steht,

oder mit Isocyanaten der Formel (IV),

$R^6-NCO$ (IV)

in welcher

$R^6$ für gegebenenfalls substituierte Reste aus der Reihe Alkyl oder Cycloalkyl steht,

gegebenenfalls in Gegenwart eines Säureakzeptors und gegebenenfalls in Gegenwart von Verdünnungsmitteln umsetzt, oder

b) Phosphorylmethylcarbonyl-Verbindungen der Formel (V),

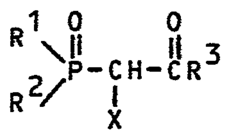

(V)

in welcher

$R^1$, $R^2$, $R^3$ und X die oben angegebenen Bedeutungen haben,

mit Chlorierungsmitteln gegebenenfalls in Gegenwart von Säureakzeptoren und gegebenenfalls in Gegenwart von Verdünnungsmitteln umsetzt.

Die neuen chlorierten Phosphorylmethylcarbonyl-Derivate der Formel (I) zeichnen sich durch hohe herbizide und fungizide Wirksamkeit aus.

Ueberraschenderweise sind die erfindungsgemäßen Wirkstoffe der Formel (I) gegen ökonomisch wichtige Ungräser in dikotylen Kulturen, wie Baumwolle, Soja, Zuckerrüben und Erdnüssen den vorbekannten chlorierten Phosphorylessigsäureestern (gemäß EP 1018) überlegen. Sie zeigen außerdem eine bessere systemische Wirkung gegen Pflanzen-

krankheiten als die bereits erwähnten Bis-thiocarbamate (gemäß US-PS 24 57 674 und 30 50 439).

Gegenstand der Erfindung sind vorzugsweise die neuen chlorierten Phosphorylmethylcarbonyl-Derivate der Formel (I), in welcher

$R^1$ für gegebenenfalls einfach oder mehrfach durch Halogen [wie insbesondere Fluor, Chlor und/oder Brom] oder $C_1$-$C_4$-Alkoxy substituierte Reste aus der Reihe Alkyl mit bis zu 6 Kohlenstoffatomen, Alkoxy mit bis zu 6 Kohlenstoffatomen, Aralkyl oder Aralkoxy mit 6 bis 10 Kohlenstoffatomen im Arylteil und bis zu 2 Kohlenstoffatomen im Alkylteil [wie insbesondere Benzyl, Phenylethyl, Benzyloxy und Phenylethoxy] oder für gegebenenfalls einfach oder mehrfach durch Halogen [wie insbesondere Fluor, Chlor und/oder Brom], Nitro, $C_1$-$C_4$-Alkyl, $C_1$-$C_4$-Alkoxy und/oder $C_1$-$C_4$-Alkoxycarbonyl substituiertes Phenyl steht,

$R^2$ für gegebenenfalls einfach oder mehrfach durch Halogen [wie insbesondere Fluor, Chlor und/oder Brom] oder $C_1$-$C_4$-Alkoxy substituiertes Alkoxy mit bis zu 6 Kohlenstoffatomen und Aralkoxy mit 6 bis 10 Kohlenstoffatomen im Arylteil und bis zu 2 Kohlenstoffatomen im Alkylteil [wie insbesondere Benzyloxy oder Phenylethoxy] steht, oder

$R^1$ und $R^2$ gemeinsam für einen verzweigten oder unverzweigten Alkandioxy-Rest mit 2 bis 5 Kohlenstoffatomen im Alkylteil stehen,

X für Wasserstoff oder Chlor steht,

$R^3$ für die Gruppierung $-OCH(R^5)-R^4$ steht,

in welcher

$R^4$ für Wasserstoff oder Alkyl mit bis zu 4 Kohlenstoffatomen steht und

$R^5$ für einen Alkoxycarbonyl-Rest mit bis zu 4 Kohlenstoffatomen im Alkylteil oder für einen gegebenenfalls substituierten Pyrazolyl-Rest

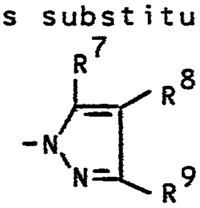

steht, wobei

$R^7$ und $R^9$ gleich oder verschieden sind und für Wasserstoff oder $C_1$-$C_4$-Alkyl stehen und

$R^8$ für Wasserstoff, Halogen [wie insbesondere Chlor] oder $C_1$-$C_4$-Alkyl steht; oder

$R^3$ für den Fall, daß X für Chlor steht, auch für gegebenenfalls einfach oder mehrfach durch Halogen, [wie insbesondere Fluor, Chlor und/oder Brom], $C_1$-$C_4$-Alkoxy und/oder $C_3$-$C_6$-Cycloalkyl substituiertes Alkyl mit bis zu 8 Kohlenstoffatomen oder für gegebenenfalls einfach oder mehrfach durch $C_1$-$C_3$-Alkyl substituiertes Cycloalkyl mit 3 bis 6 Kohlenstoffatomen, Alkylamino mit bis zu 6 Kohlenstoffatomen im Alkylteil und Cycloalkylamino mit 3 bis 6 Kohlenstoffatomen steht.

Die Erfindung betrifft insbesondere Verbindungen der Formel (I), in welcher

(A) $R^1$ für n-Propoxy, i-Propoxy, n-Butoxy, i-Butoxy, sec.-Butoxy oder tert.-Butoxy steht,

$R^2$ für n-Propoxy, i-Propoxy, n-Butoxy, i-Butoxy,

sec.-Butoxy oder tert.-Butoxy steht,

X für Chlor steht und

$R^3$ für n-Propyl, i-Propyl, n-Butyl, i-Butyl, sec.-Butyl, tert.-Butyl, Cyclopropyl, n-Propylamino, i-Propylamino,n-Butylamino, i-Butylamino, sec.-Butylamino oder tert.-Butylamino steht,

oder

(B) $R^1$ für n-Propoxy, i-Propoxy, n-Butoxy, i-Butoxy, sec.-Butoxy, tert.-Butoxy oder Phenyl steht,

$R^2$ für n-Propoxy, i-Propoxy, n-Butoxy, i-Butoxy, sec.-Butoxy oder tert.-Butoxy steht,

X für Chlor steht und

$R^3$ für die Gruppierung $-OCH\genfrac{}{}{0pt}{}{R^4}{R^5}$ steht,

in welcher

$R^4$ für Methyl oder Ethyl steht und

$R^5$ für Methoxycarbonyl, Ethoxycarbonyl, n-Propoxycarbonyl, i-Propoxycarbonyl, n-Butoxycarbonyl, i-Butoxycarbonyl, sec.-Butoxycarbonyl oder tert.-Butoxycarbonyl steht,

oder

(C) $R^1$ für n-Propoxy, i-Propoxy, n-Butoxy, i-Butoxy, sec.-Butoxy, tert.-Butoxy oder Phenyl steht,

$R^2$ für n-Propoxy, i-Propoxy, n-Butoxy, i-Butoxy, sec.-Butoxy oder tert.-Butoxy steht,

X für Wasserstoff oder Chlor steht und

$R^3$ für die Gruppierung 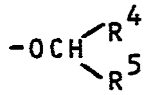 steht,

in welcher

$R^4$ für Wasserstoff, Methyl, Ethyl, n-Propyl oder i-Propyl steht und

$R^5$ für einen gegebenenfalls substituierten Pyrazolyl-Rest

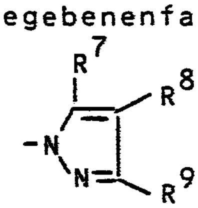

steht, wobei

$R^7$ und $R^9$ gleich oder verschieden sind und für Wasserstoff oder Methyl stehen und

$R^8$ für Wasserstoff oder Chlor steht.

Verwendet man beispielsweise O,O-Di-n-propyl-dichlormethanphosphonsäureester und Cyclohexylcarbonsäurechlorid bzw. Cyclohexylisocyanat als Ausgangsstoffe, so kann der Reaktionsablauf nach der erfindungsgemäßen Verfahrensvariante (a) durch folgendes Formelschema wiedergegeben werden :

(a) 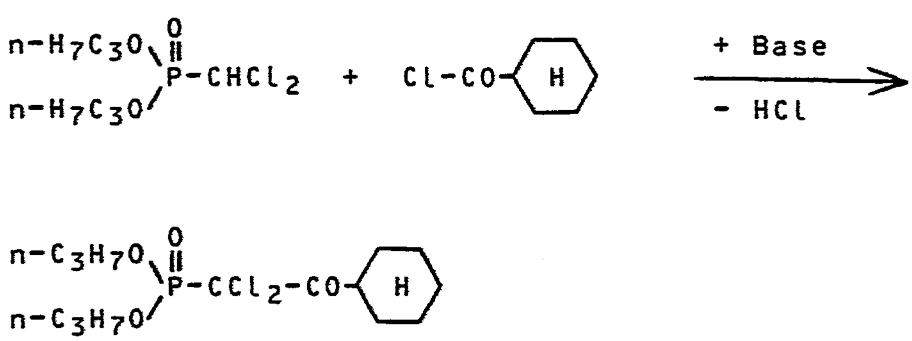

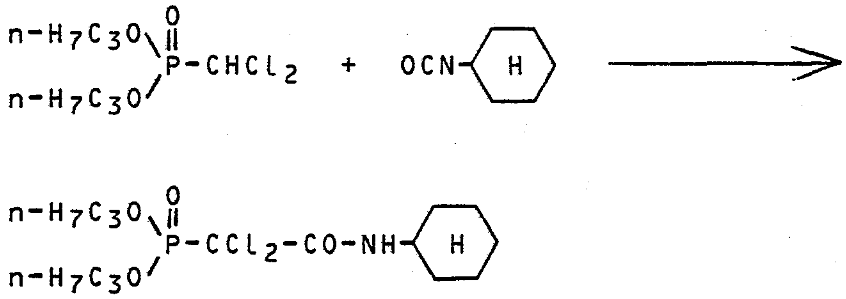

Verwendet man beispielsweise Natriumhypochlorit als Chlorierungsmittel und O,O-Di-n-propoxy-phosphoryl-essigsäure-1-(1-pyrazol-1-yl)-ethylester als Ausgangsstoff, so kann der Reaktionsablauf nach der erfindungsgemäßen Verfahrensvariante (b) durch folgendes Formelschema wiedergegeben werden :

(b)

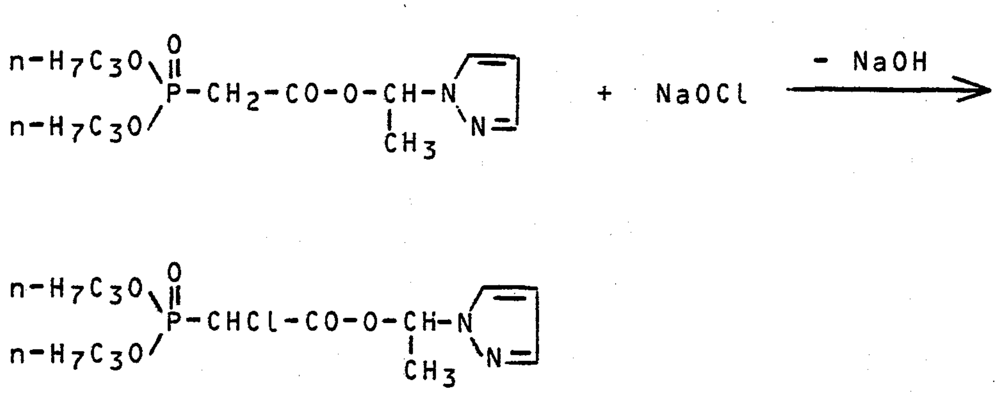


Die als Ausgangsstoffe zu verwendenden Dichlormethanphosphonsäure-Derivate sind durch Formel (II) definiert. In dieser Formel stehen $R^1$ und $R^2$ für gegebenenfalls substituierte Reste aus der Reihe Alkoxy oder Aralkoxy oder stehen gemeinsam für einen Alkandioxy-Rest. Die bevorzugte bzw. besonders bevorzugte Definition dieser Reste ist bereits im Rahmen der Substituentendefinition der Formel (I) genannt worden.

Als Beispiele für die Verbindungen der Formel (II) seien genannt :

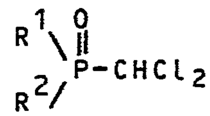

(II)

Tabelle 1 :

| $R^1$ | $R^2$ | $R^1$ | $R^2$ |
|---|---|---|---|
| $-OC_3H_7$-n | $-OC_3H_7$-n | $-OC_3H_7$-n | $-OC_4H_9$-iso |
| $-OC_3H_7$-iso | $-OC_3H_7$-iso | $-OC_3H_7$-n | $-OC_4H_9$-sec. |
| $-OC_4H_9$-n | $-OC_4H_9$-n | $-OC_3H_7$-n | $-OC_4H_9$-tert. |
| $-OC_4H_9$-iso | $-OC_4H_9$-iso | $-OC_3H_7$-i | $-OC_4H_9$-n |
| $-OC_4H_9$-sec. | $-OC_4H_9$-sec. | $-OC_3H_7$-i | $-OC_4H_9$-iso |
| $-OC_4H_9$-tert. | $-OC_4H_9$-tert. | $-OC_3H_7$-i | $-OC_4H_9$-sec. |
| $-OCH_3$ | $-OC_3H_7$-n | $-OC_3H_7$-i | $-OC_4H_9$-tert. |
| $-OCH_3$ | $-OC_3H_7$-iso | $-OC_4H_9$-n | $-OC_4H_9$-sec. |
| $-OCH_3$ | $-OC_4H_9$-n | $-OC_4H_9$-n | $-OC_4H_9$-tert. |
| $-OCH_3$ | $-OC_4H_9$-iso | $-OC_4H_9$-iso | $-OC_4H_9$-sec. |
| $-OCH_3$ | $-OC_4H_9$-sec. | $-OC_4H_9$-iso | $-OC_4H_9$-tert. |
| $-OCH_3$ | $-OC_4H_9$-tert. | $-O-CH_2CH_2-O-$ | |
| $-OC_2H_5$ | $-OC_3H_7$-n | $-O-CH_2CH_2CH_2-O-$ | |
| $-OC_2H_5$ | $-OC_3H_7$-iso | 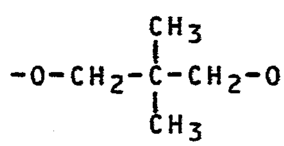 | |
| $-OC_2H_5$ | $-OC_4H_9$-n | | |
| $-OC_2H_5$ | $-OC_4H_9$-iso | 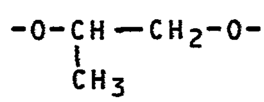 | |
| $-OC_2H_5$ | $-OC_4H_9$-sec. | | |
| $-OC_2H_5$ | $-OC_4H_9$-tert. | 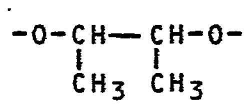 | |
| $-OC_3H_7$-n | $-OC_3H_7$-iso | | |
| $-OC_3H_7$-n | $-OC_4H_9$-n | | |

Die Ausgangsstoffe der Formel (II) sind bekannt und/oder können nach an sich bekannten Verfahren hergestellt werden (vgl. Tetrahedron Letters 1975, 609-610).

Die weiterhin als Ausgangsstoffe für die Verfahrensvariante (a) zu verwendenden Säurehalogenide sind durch die Formel (III) definiert. In dieser Formel steht $R^3$ vorzugsweise bzw. besonders bevorzugt für diejenigen Reste, welche bereits im Rahmen der Substituentendefinitionen der Formel (I) vorzugsweise bzw. als insbesondere bevorzugt genannt sind; Hal steht für Chlor oder Brom.

Als Ausgangsstoffe der Formel (III) seien beispielsweise genannt : Buttersäure-, Isobuttersäure-, Valeriansäure-, Isovaleriansäure-, Capronsäure- und Cyclopropancarbonsäurechlorid bzw. -bromid.

Die Säurehalogenide der Formel (III) sind bekannt und/oder können nach üblichen Methoden hergestellt werden.

Für die Verfahrensvariante (a) können außerdem die Isocyanate der Formel (IV) als Ausgangsstoffe verwendet werden. In dieser Formel steht $R^6$ vorzugsweise für Alkyl mit 3 bis 6 Kohlenstoffatomen oder für Cycloalkyl mit 3 bis 6 Kohlenstoffatomen. Besonders bevorzugt steht $R^6$ für Alkyl mit 3 bis 6 Kohlenstoffatomen.

Als Ausgangsstoffe der Formel (IV) seien beispielsweise genannt : n-Propyl-, i-Propyl-, n-Butyl-, i-Butyl-, sec.-Butyl- und tert.-Butyl-isocyanat.

Die Isocyanate der Formel (IV) sind allgemein bekannte Verbindungen der organischen Chemie.

Die als Ausgangsstoffe für die Verfahrensvariante (b) zu verwendenden Phosphorylmethylcarbonyl-Verbindungen sind durch die Formel (V) definiert. In dieser Formel stehen $R^1$, $R^2$, $R^3$ und X vorzugsweise bzw. insbesondere bevorzugt für diejenigen Reste, welche bereits im Rahmen der Substituentendefinition der Formel (I) vorzugsweise bzw. als insbesondere bevorzugt genannt sind.

Als Ausgangsstoffe der Formel (V) seien beispielsweise folgende Verbindungen genannt:

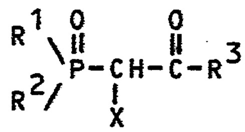 (V)

Tabelle 2 :

| $R^1$ | $R^2$ | X | $R^3$ |
|---|---|---|---|
| $-OC_3H_7-i$ | $-OC_3H_7-i$ | H | 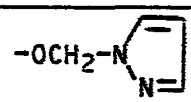 |
| $-OC_3H_7-i$ | $-OC_3H_7-i$ | H | 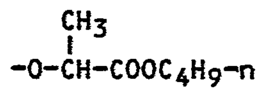 |
| $-OC_3H_7-i$ | $-OC_3H_7-i$ | H | 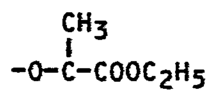 |
| $-OC_3H_7-i$ | $-OC_3H_7-i$ | H | 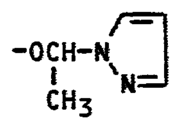 |
| 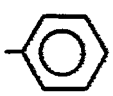 | $-OC_3H_7-i$ | H | 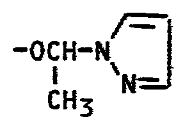 |
| 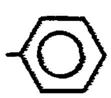 | $-OC_3H_7-i$ | H | 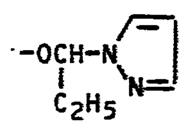 |

Tabelle 2 - Fortsetzung :

| $R^1$ | $R^2$ | X | $R^3$ |
|---|---|---|---|
| $-OC_3H_7-i$ | $-OC_3H_7-i$ | H | 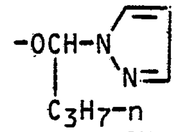 |
| $-OC_3H_7-i$ | $-OC_3H_7-i$ | H | 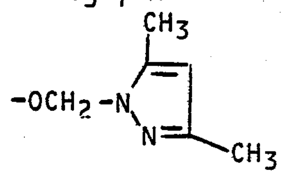 |
| $-OC_3H_7-i$ | $-OC_3H_7-i$ | H | $-CH_2CH(CH_3)_2$ |
| $-OC_3H_7-i$ | $-OC_3H_7-i$ | H | 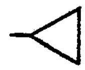 |
| $-OC_3H_7-i$ | $-OC_3H_7-i$ | H | $-OCH_2-COOC_2H_5$ |
| $-OC_3H_7-i$ | $-OC_3H_7-i$ | H | $-OCH_2-COOC_4H_9-n$ |
| $-OC_3H_7-n$ | $-OC_3H_7-n$ | Cl | $-CH_2CH(CH_3)_2$ |
| $-OC_3H_7-i$ | $-OC_3H_7-i$ | Cl | $-CH(CH_3)_2$ |
| $-OC_4H_9-i$ | $-OC_4H_9-i$ | Cl | $-CH_2CH(CH_3)_2$ |
| $-OC_3H_7-i$ | $-OC_3H_7-i$ | H | $-CH(CH_3)_2$ |
| $-OC_4H_9-sec.$ | $-OC_4H_9-sec.$ | Cl | $-CH_2CH(CH_3)_2$ |
| $-OC_3H_7-i$ | $-OC_3H_7-i$ | Cl | $-C_3H_7-n$ |
| $-OC_3H_7-i$ | $-OC_3H_7-i$ | Cl | $-CH_2CH(CH_3)_2$ |
| $-OC_3H_7-i$ | $-OC_3H_7-i$ | H | $-C_3H_7-n$ |
| $-OC_4H_9-i$ | $-OC_4H_9-i$ | H | $-CH_2CH(CH_3)_2$ |
| $-OC_3H_7-i$ | $-OC_3H_7-i$ | H | 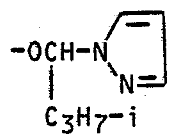 |
| $-OC_3H_7-i$ | $-OC_3H_7-i$ | H | 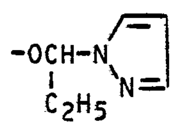 |
| 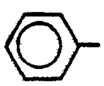 | $-OC_3H_7-i$ | H | $-OCH_2COOC_2H_5$ |
| 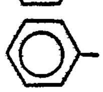 | $-OC_3H_7-i$ | H | $-OCH_2COOC_4H_9-n$ |
| 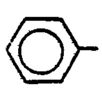 | $-OC_3H_7-i$ | H | $-OCHCOOC_2H_5$<br>$CH_3$ |

Tabelle 2 - Fortsetzung :

| $R^1$ | $R^2$ | X | $R^3$ |
|---|---|---|---|
| 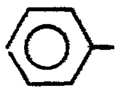 | $-OC_3H_7-i$ | H | 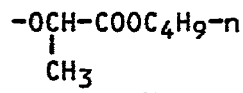 |
| 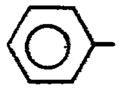 | $-OC_3H_7-i$ | H | 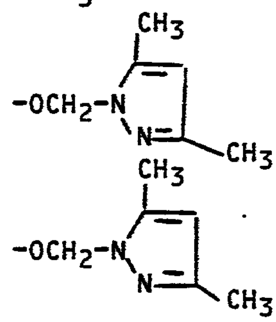 |
| $-OC_4H_9-n$ | $-OC_4H_9-n$ | H | |
| $-OC_4H_9-n$ | $-OC_4H_9-n$ | H | 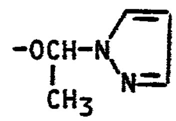 |
| $-OC_4H_9-n$ | $-OC_4H_9-n$ | H | 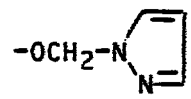 |
| $-OC_4H_9-n$ | $-OC_4H_9-n$ | H | 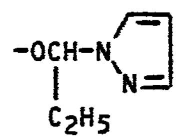 |
| $-OC_4H_9-n$ | $-OC_4H_9-n$ | H | 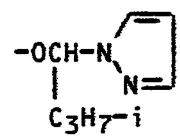 |
| $-OC_3H_7-n$ | $-OC_3H_7-n$ | H | 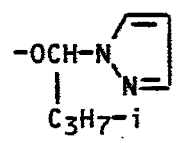 |
| $-OC_4H_9-n$ | $-OC_4H_9-n$ | H | $-OCH_2COOC_2H_5$ |
| $-OC_3H_7-n$ | $-OC_3H_7-n$ | H | $-OCH_2COOC_2H_5$ |
| $-OC_4H_9-n$ | $-OC_4H_9-n$ | H | $-OCH_2COOC_4H_9-n$ |
| $-OC_3H_7-n$ | $-OC_3H_7-n$ | H | $-OCH_2COOC_4H_9-n$ |
| $-OC_3H_7-n$ | $-OC_3H_7-n$ | Cl | $-CH(CH_3)_2$ |

Tabelle 2 - Fortsetzung :

| $R^1$ | $R^2$ | X | $R^3$ |
|---|---|---|---|
| $-OC_3H_7-n$ | $-OC_3H_7-n$ | H | $-CH(CH_3)_2$ |
| $-OC_3H_7-n$ | $-OC_3H_7-n$ | Cl | $-C_3H_7-n$ |
| $-OC_3H_7-n$ | $-OC_3H_7-n$ | H | $-C_3H_7-n$ |

Die Ausgangsverbindungen der Formel (V) sind zum Teil noch nicht in der Literatur beschrieben. Neu sind beispielsweise die Verbindungen der Formel (Va),

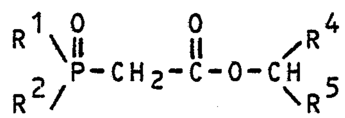

(Va)

in welcher

$R^1$, $R^2$, $R^4$ und $R^5$ die oben angegebenen Bedeutungen haben.

Man erhält die neuen Verbindungen der Formel (Va), wenn man Verbindungen der Formel (VI),

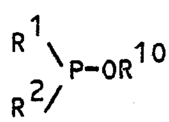

(VI)

in welcher

$R^1$ und $R^2$ die oben genannten Bedeutungen haben und

$R^{10}$ für gegebenenfalls substituiertes $C_1$-$C_4$-Alkyl oder Benzyl steht, .

mit Halogenessigsäureestern der Formel (VII),

$$Hal^1-CH_2-CO-O-CH\begin{matrix}R^4\\R^5\end{matrix} \qquad (VII)$$

in welcher

$R^4$ und $R^5$ die oben genannten Bedeutungen haben und

$Hal^1$ für Halogen, insbesondere für Chlor oder Brom steht,

auf Temperaturen zwischen 50°C und 200°C, vorzugsweise zwischen 100°C und 150°C erhitzt und die Reaktionsprodukte der Formel $Hal^1-R^{10}$, gegebenenfalls unter vermindertem Druck, entfernt.

Die Verbindungen der Formel (VI) sind allgemein bekannte Verbindungen der organischen Chemie.

Als Beispiele seien genannt : Phosphorigsäure-tri-n-propylester, -tri-i-propylester, -tri-n-butylester, -tri-i-butylester und -tri-sec.-butylester sowie Phenylphosphonigsäure-di-n-propylester, -di-i-propylester, -di-n-butylester, -di-i-butylester und -di-sec.-butylester.

Die Halogenessigsäureester der Formel (VII) sind neu. Man erhält die Verbindungen der Formel (VII) durch Umsetzung von Halogenessigsäurehalogeniden der Formel (VIII),

$$Hal^1-CH_2-CO-Hal^2 \qquad (VIII)$$

in welcher

$Hal^1$ und $Hal^2$ für Halogen, insbesondere für Chlor oder Brom stehen,

mit Hydroxy-Derivaten der Formel (IX),

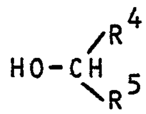

(IX)

in welcher

$R^4$ und $R^5$ die oben angegebenen Bedeutungen haben,

in Gegenwart von Säureakzeptoren, wie z.B. Triethylamin, und in Gegenwart von Verdünnungsmitteln, wie z.B. Methylenchlorid bei Temperaturen zwischen -50°C und +100°C, vorzugsweise zwischen -20°C und +50°C.

Die Aufarbeitung kann nach üblichen Methoden erfolgen, beispielsweise durch Waschen der organischen Lösung mit wässriger Natriumhydrogencarbonat-Lösung, Trocknen, Filtrieren der organischen Phase und Abdestillieren des Lösungsmittels unter vermindertem Druck.

Die Ausgangsverbindungen der Formel (VIII) sind allgemein bekannte Verbindungen der organischen Chemie.

Die Hydroxy-Derivate der Formel (IX) sind bereits bekannt (vgl. z.B. DE 28 35 158).

Die Verfahrensvariante (a) wird vorzugsweise unter Verwendung von Verdünnungsmitteln durchgeführt.

Als Verdünnungsmittel kommen praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methyl-isopropyl- und Methyl-isobutylketon, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon, Hexamethylphosphorsäuretriamid und Pyridin.

Die Verfahrensvariante (a) wird vorzugsweise in Gegenwart von Säureakzeptoren durchgeführt. Hierzu gehören vorzugsweise Alkalimetall- und Erdalkalimetall-hydride, wie z.B. Natrium- und Calcium-hydrid, metallorganische Verbindungen, wie z.B. Butyllithium oder Isopropylmagnesiumchlorid, Alkoholate, wie z.B. Kalium-tert.-butanolat und Aluminiumisopropylat sowie spezielle Amine, wie z.B. Diazabicyclooctan, Diazabicycloundecen und Pyridin.

Die Reaktionstemperatur kann bei Verfahren (a) zur Herstellung der Verbindungen der Formel (I) innerhalb eines größeren Bereiches variiert werden. Im allgemeinen arbeitet man zwischen -120°C und +100°C, vorzugsweise bei

-70°C bis +50°C. Das Verfahren (a) wird im allgemeinen bei Normaldruck durchgeführt.

Zur Durchführung des Verfahrens (a) setzt man im allgemeinen äquimolare Mengen der Ausgangsstoffe der Formeln (II) und (III) bzw. (II) und (IV) und zusätzlich 1 bis 2, vorzugsweise 1,0 bis 1,4 Moläquivalente einer geeigneten Base ein.

Im allgemeinen werden das Dichlormethanphosphonsäure-Derivat der Formel (II) und die Base in einem geeigneten Lösungsmittel, gegebenenfalls unter Kühlen, zusammengegeben und das Säurehalogenid der Formel (III) bzw. das Isocyanat der Formel (IV) wird, gegebenenfalls in Lösung, langsam dazugegeben. Das Reaktionsgemisch wird bis zum Ende der Umsetzung gerührt. Die Aufarbeitung kann nach üblichen Methoden durchgeführt werden, beispielsweise durch Ansäuern, z.B. mit Schwefelsäure, Extraktion mit Methylenchlorid, Trocknen und Filtrieren der organischen Bestandteile bei vermindertem Druck und mäßig erhöhter Temperatur. Die schwerflüchtigen Rückstände enthalten im wesentlichen die Verbindungen der Formel (I), welche durch ihre physikalisch-chemischen Eigenschaften charakterisiert werden.

Das Verfahren (b) wird unter Verwendung von Chlorierungsmitteln durchgeführt. Als solche sind Chlor, Sulfurylchlorid und Alkalimetall- oder Erdalkalimetall-hypochlorite, wie z.B. Natriumhypochlorit (gegebenenfalls in Form der sogenannten Chlorlauge) oder Calcium-hypochlorit zu nennen. Vorzugsweise wird Chlorlauge verwendet.

Verfahren (b) wird vorzugsweise in Gegenwart von Verdünnungsmitteln durchgeführt. Als solche kommen insbesondere halogenierte Kohlenwasserstoffe, wie z.B. Methylenchlorid, Ethylenchlorid, Chloroform oder Tetrachlormethan in Betracht. Bei Einsatz von Chlorlauge wird Wasser als Verdünnungsmittel verwendet, das gegebenenfalls durch ein inertes organisches Lösungsmittel ergänzt wird.

Bei Verfahren (b) werden gegebenenfalls Säureakzeptoren verwendet. Bei Einsatz von wässrigen Hypochlorit-Lösungen dienen vorzugsweise entsprechende Alkalihydroxide oder Erdalkalihydroxide, wie z.B. Natrium-, Kalium- oder Calcium-hydroxid, als Säurebindemittel.

Die Reaktionstemperaturen können bei Verfahren (b) innerhalb eines größeren Bereiches variiert werden. Im allgemeinen arbeitet man zwischen -20°C und +50°C, vorzugsweise bei 0°C bis 30°C. Das Verfahren wird im allgemeinen bei Normaldruck durchgeführt.

Zur Durchführung von Verfahren (b) werden im allgemeinen je Mol Phosphorylmethylcarbonyl-Verbindung der Formel (V) zwischen 0,5 und 5, vorzugsweise zwischen 0,9 und 3,5 Moläquivalente Chlorierungsmittel eingesetzt und der pH-Wert wird im allgemeinen zwischen 7 und 14, vorzugsweise zwischen 9 und 14 gehalten.

Im allgemeinen werden Chlorierungsmittel und das Verdünnungsmittel vorgelegt und die Verbindung der Formel (V) wird unter starkem Rühren dazugegeben. Nach Ende der Umsetzung kann nach üblichen Methoden aufgearbeitet werden, beispielsweise durch Waschen der organischen Lö-

sung mit wässriger Natriumhydrogencarbonatlösung, Trocknen und Filtrieren der organischen Phase und Abdestillieren des Lösungsmittels unter vermindertem Druck bei mäßig erhöhter Temperatur. Die schwerflüchtigen Rückstände enthalten im wesentlichen die Verbindungen der Formel (I), welche durch ihre physikalisch-chemischen Eigenschaften charakterisiert werden.

Die erfindungsgemäßen Wirkstoffe der Formel (I) können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten auchwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe weisen außerdem eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur systemischen Bekämpfung von Reiskrankheiten, wie Pyricularia oryzae eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen, Tauchen, Vernebeln, Verdampfen, Injizieren, Verschlämmen, Verstreichen, Stäuben, Trockenbeizen, Feuchtbeizen, Naßbeizen, Schlämmbeizen oder Inkrustieren.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,05 und 10 kg Wirkstoff pro ha, vorzugsweise zwischen 0,1 und 5 kg pro ha.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise 0,0001 bis 0,02 Gew.-%, am Wirkungsort erforderlich.

Die nachfolgenden Beispiele dienen zur weiteren Erläuterung der Erfindung.

## Herstellungsbeispiele :

### Beispiel 1 :

$$(iso\text{-}H_7C_3O)_2\overset{O}{\overset{\|}{P}}\text{-}CCl_2\text{-}CO\text{-}CH_2\text{-}CH(CH_3)_2$$

(Verfahrensvariante a)

37,4 g (0,15 Mol) Dichlormethanphosphonsäurediisopropylester werden in 100 ml Tetrahydrofuran vorgelegt, auf -70°C gekühlt und mit 100 ml einer 15 %-igen Lösung von Butyllithium in n-Hexan versetzt. Das Gemisch wird eine Stunde bei -70°C gerührt, mit 18,1 g (0,15 Mol) Isovaleriansäurechlorid versetzt und eine weitere Stunde bei -70°C gerührt. Bei einer Temperatur von 0°C werden langsam 50 ml 1n Schwefelsäure dazugetropft; man extrahiert anschließend dreimal mit Methylenchlorid, trocknet und filtriert die organische Phase und destilliert das Lösungsmittel unter vermindertem Druck ab.

Man erhält 34,8 g (69 % der Theorie) 1,1-Dichlor-1-(O,O-di-i-propyl-phosphoryl)-4-methyl-pentan-2-on vom Brechungsindex $n_D^{20}$ : 1,4455.

Beispiel 2 :

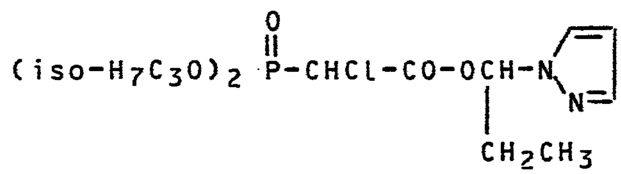

(Verfahrensvariante b)

28 g Chlorlauge (mit 11,9 % 'aktivem Chlor') werden in 100 ml Tetrachlormethan vorgelegt und auf 0°C bis 5°C gekühlt. Dazu werden 13,3 g (0,04 Mol) 1-(O,O-Di-i-propyl-phosphoryl)-essigsäure-1-(pyrazol-1-yl)-n-propylester in 50 ml Tetrachlormethan unter heftigem Rühren getropft. Das Gemisch wird 2 Stunden bei 0°C bis 5°C nachgerührt, mit Natriumhydrogencarbonatlösung gewaschen, die organische Phase über Natriumsulfat getrocknet, filtriert, eingeengt und 10 Minuten bei 70°C am Dampfstrahler andestilliert.

Man erhält 6,7 g (46 % der Theorie) 1-Chlor-1-(O,O-di-i-propyl-phosphoryl)-essigsäure-1-(pyrazol-1-yl)-n-propylester in Form eines Oels vom Brechungsindex $n_D^{20}$ : 1,4557.

Analog Beispiel 1 und 2 bzw. analog Verfahrensvariante (a) oder (b) wurden die in der nachfolgenden Tabelle 3 aufgeführten Verbindungen der Formel (I) hergestellt :

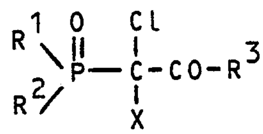

(I)

Tabelle 3 :

| Beisp. Nr. | $R^1$ | $R^2$ | X | $R^3$ | Brechungsindex $n_D^{20}$ |
|---|---|---|---|---|---|
| 3 | $-OC_3H_7$-iso | $-OC_3H_7$-iso | Cl | $-CH(CH_3)_2$ | 1,4367 |
| 4 | $-OC_3H_7$-iso | $-OC_3H_7$-iso | Cl | $-NH-CH(CH_3)_2$ | 1,4550 |
| 5 | $-OC_3H_7$-iso | $-OC_3H_7$-iso | Cl | 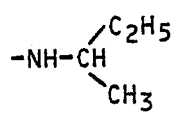 | 1,4546 |
| 6 | $-OC_3H_7$-iso | $-OC_3H_7$-iso | Cl | 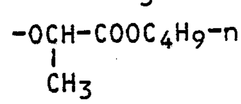 | 1,4385 |
| 7 | $-OC_3H_7$-iso | $-OC_3H_7$-iso | Cl | 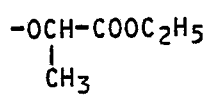 | 1,4432 |
| 8 | $-OC_3H_7$-iso | $-OC_3H_7$-iso | Cl | 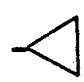 | 1,4611 |
| 9 | $-OC_3H_7$-iso | $-OC_3H_7$-iso | Cl | 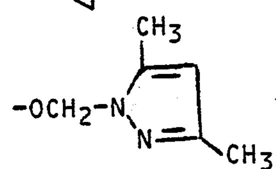 | 1,4681 |
| 10 | $-OC_3H_7$-iso | $-OC_3H_7$-iso | H | 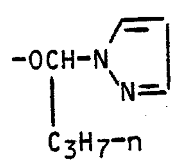 | 1,4658 |
| 11 | 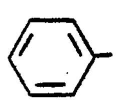 | $-OC_3H_7$-iso | Cl | 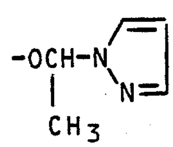 | 1,5265 |

Tabelle 3 - Fortsetzung :

| Beisp. Nr. | $R^1$ | $R^2$ | X | $R^3$ | Brechungsindex $n_D^{20}$ |
|---|---|---|---|---|---|
| 12 | $-OC_3H_7$-iso | $-OC_3H_7$-iso | H | 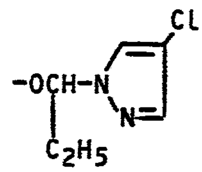 | 1,4711 |
| 13 | $-OC_3H_7$-iso | $-OC_3H_7$-iso | H | 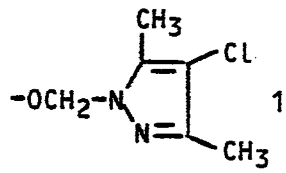 | 1,4795 |
| 14 | $-OC_3H_7$-iso | $-OC_3H_7$-iso | Cl | 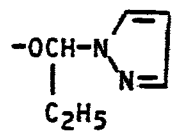 | 1,4585 |
| 15 | $-OC_4H_9$-n | $-OC_4H_9$-n | H | 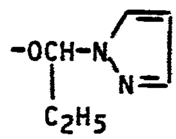 | 1,4618 |
| 16 | $-OC_4H_9$-n | $-OC_4H_9$-n | Cl | 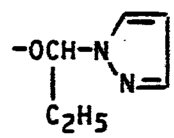 | 1,4648 |
| 17 | $-OC_4H_9$-iso | $-OC_4H_9$-iso | Cl | $-CH_2CH(CH_3)_2$ | |
| 18 | $-OC_4H_9$-sec. | $-OC_4H_9$-sec. | Cl | $-CH_2CH(CH_3)_2$ | |
| 19 | 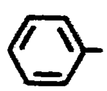 | $-OC_3H_7$-iso | H | 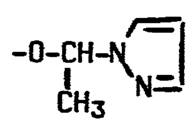 | 1,5295 |
| 20 | $-OC_3H_7$-iso | $-OC_3H_7$-iso | H | 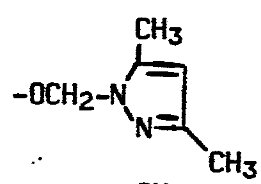 | 1,4689 |
| 21 | $-OC_4H_9$-n | $-OC_4H_9$-n | H | 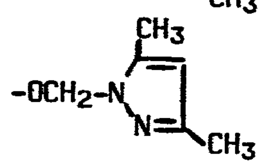 | 1,4694 |

Tabelle 3 - Fortsetzung

| Beisp. Nr. | $R^1$ | $R^2$ | X | $R^3$ | Brechungsindex $n_D^{20}$ |
|---|---|---|---|---|---|
| 22 | 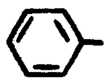 | $-OC_3H_7$-iso | Cl | 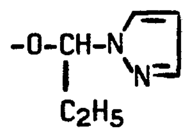 | 1,5183 |
| 23 | $-OCH_2CH_2Cl$ | $-OCH_2CH_2Cl$ | Cl | 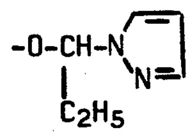 | 1,5009 |

Herstellung der Ausgangsstoffe der Formel (Va) :

Beispiel (Va-1) :

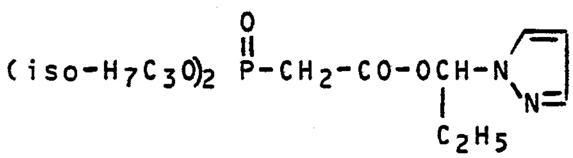

Eine Mischung aus 16,2 g (0,08 Mol) 1-Chlor-essigsäure-1-(pyrazol-1-yl)-1-n-propylester und 18,4 g (0,088 Mol) Tri-isopropylphosphit wird 4 Stunden bei 140°C gerührt und dann 10 Minuten bei 70°C am Dampfstrahler andestilliert.

Man erhält 22,7 g (85 % der Theorie) 1-(O,O-Di-isopropyl-phosphoryl)-essigsäure-1-(pyrazol-1-yl)-n-propylester mit einem Brechungsindex $n_D^{20}$ : 1,4567.

Analog Beispiel (Va-1) wurden die übrigen in Tabelle 4 aufgeführten Verbindungen der Formel (Va) hergestellt :

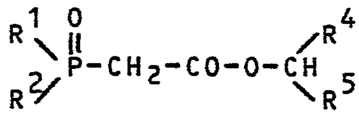

(Va)

Tabelle 4 :

| Beisp. Nr. | $R^1$ | $R^2$ | $R^4$ | $R^5$ | Brechungsindex $n_D^{20}$ |
|---|---|---|---|---|---|
| Va-2 | $-OC_3H_7$-iso | $-OC_3H_7$-iso | $CH_3$ | $-COOC_4H_9$-n | |
| Va-3 | $-OC_3H_7$-iso | $-OC_3H_7$-iso | $CH_3$ | $-COOC_2H_5$ | |
| Va-4 | $-OC_3H_7$-iso | $-OC_3H_7$-iso | $-C_3H_7$-iso | 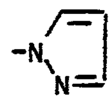 | 1,4538 |
| Va-5 | $-OC_3H_7$-iso | $-OC_3H_7$-iso | H | 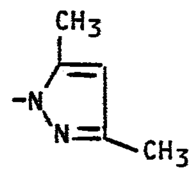 | 1,4635 |
| Va-6 | $-OC_3H_7$-iso | $-OC_3H_7$-iso | $-C_3H_7$-n | 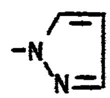 | 1,4595 |
| Va-7 | $-OC_3H_7$-iso | $-OC_3H_7$-iso | $CH_3$ | 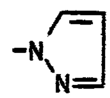 | 1,4539 |
| Va-8 | 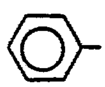 | $-OC_3H_7$-iso | $CH_3$ | $-COOC_2H_5$ | 1,4818 |
| Va-9 | 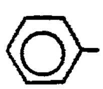 | $-OC_3H_7$-iso | $CH_3$ | 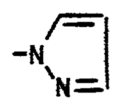 | 1,4293 |
| Va-10 | 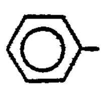 | $-OC_3H_7$-iso | $CH_3$ | $-COOC_4H_9$-n | 1,4765 |
| Va-11 | $-OC_3H_7$-iso | $-OC_3H_7$-iso | H | 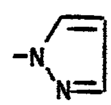 | 1,4579 |

Tabelle 4 - Fortsetzung :

| Beisp. Nr. | $R^1$ | $R^2$ | $R^4$ | $R^5$ | Brechungsindex $n_D^{20}$ |
| --- | --- | --- | --- | --- | --- |
| Va-12 | 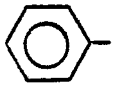 | $-OC_3H_7$-iso | H | 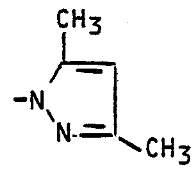 | 1,5229 |
| Va-13 | $-OC_4H_9$-n | $-OC_4H_9$-n | H | 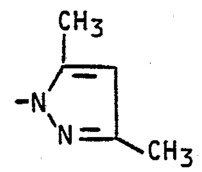 | 1,4673 |
| Va-14 | $-OC_4H_9$-n | $-OC_4H_9$-n | $-C_2H_5$ | 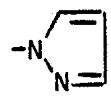 | 1,4618 |
| Va-15 | 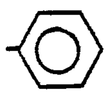 | $-OC_3H_7$-iso | $-C_2H_5$ | 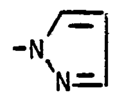 | |

Herstellung der Ausgangsstoffe der Formel (VII) :

Beispiel (VII-1) :

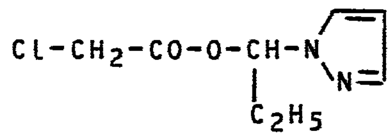

12,6 g (0,1 Mol) 1-Hydroxy-1-(pyrazol-1-yl)-propan werden in 100 ml Methylenchlorid vorgelegt, mit Eis auf 0°C bis 10°C gekühlt, 13,3 g (0,13 Mol) Triethylamin dazugegeben und anschließend 11,3 g (0,10 Mol) Chloracetylchlorid langsam und unter starkem Rühren bei 0°C bis 10°C zugetropft. Die Mischung wird 15 bis 20 Minuten nachgerührt, dreimal mit Natriumhydrogencarbonatlösung gewaschen, getrocknet, eingeengt und am Dampfstrahler bei 20°C andestilliert.

Man erhält 11,3 g (56 % der Theorie) 1-Chlor-essigsäure-1-(pyrazol-1-yl)-n-propylester vom Brechungsindex $n_D^{20}$ : 1,4875.

Analog Beispiel (VII-1) wurden die übrigen in Tabelle 5 aufgeführten Verbindungen der Formel (VII) erhalten :

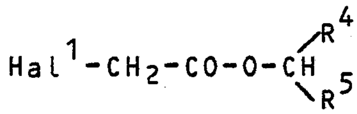

(VII)

Tabelle 5 :

| Beisp. Nr. | $Hal^1$ | $R^4$ | $R^5$ | physikalische Konstante |
|---|---|---|---|---|
| VII-2 | Cl | $CH_3$ | 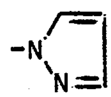 | $n_D^{20}$ : 1,4962 |
| VII-3 | Cl | H | 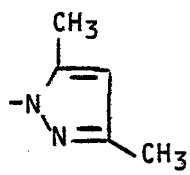 | Fp. : 58°C |
| VII-4 | Cl | $CH_3$ | $-COOC_2H_5$ | |
| VII-5 | Cl | $CH_3$ | $-COOC_4H_9$ | |
| VII-6 | Cl | $n-C_3H_7$ | 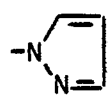 | $n_D^{20}$ : 1,4920 |
| VII-7 | Cl | $iso-C_3H_7$ | 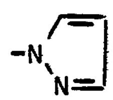 | $n_D^{20}$ : 1,4869 |
| VII-8 | Cl | H | 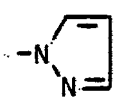 | |
| VII-9 | Br | $CH_3$ | 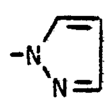 | |
| VII-10 | Br | H | 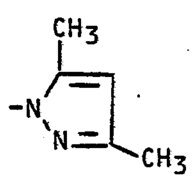 | |
| VII-11 | Br | $C_2H_5$ | 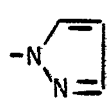 | |

0126249

Herstellung der Ausgangsstoffe der Formel (IX) :

Beispiel (IX-1) :

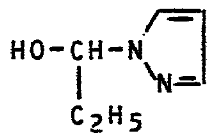

34 g (0,5 Mol) Pyrazol werden in 250 ml Methylenchlorid vorgelegt und bei 0°C bis 5°C 47 g (0,65 Mol) Propionaldehyd zugetropft. Das Gemisch wird ca. 72 Stunden gerührt und im Wasserstrahlvakuum bei 20°C andestilliert.

Man erhält 42 g (67 % der Theorie) 1-Hydroxy-1-(pyrazol-1-yl)-n-propan als farbloses Oel.

Analog Beispiel (IX-1) wurden die folgenden in Tabelle 6 aufgeführten Verbindungen der Formel (IX) erhalten :

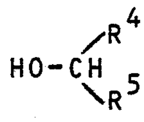 (IX)

Tabelle 6 :

| Beisp. Nr. | $R^4$ | $R^5$ | physikalische Konstante |
|---|---|---|---|
| IX-2 | $CH_3$ | 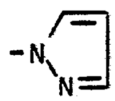 | |
| IX-3 | H | 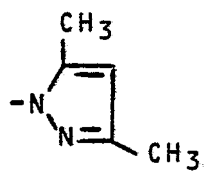 | 88°C |
| IX-4 | n-$C_3H_7$ | 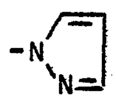 | |
| IX-5 | iso-$C_3H_7$ | 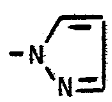 | |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die folgende Verbindung gemäß den Herstellungsbeispielen eine ausgezeichnete Wirksamkeit: (1).

0126249

Beispiel B

Pre-emergence-Test - Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die folgende Verbindung gemäß den Herstellungsbeispielen eine ausgezeichnete Wirksamkeit: (9).

Beispiel C

Pyricularia-Test (Reis) /~~xxxxxxxxx~~ /~~xxxxxxx~~ /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: (6), (7) und (8).

Patentansprüche:

1) Chlorierte Phosphorylmethylcarbonyl-Derivate der allgemeinen Formel I

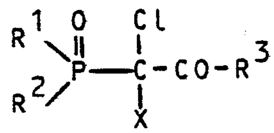 (I)

in welcher

$R^1$ für gegebenenfalls substituierte Reste aus der Reihe Alkyl, Aryl, Aralkyl, Alkoxy und Aralkoxy steht,

$R^2$ für gegebenenfalls substituierte Reste aus der Reihe Alkoxy oder Aralkoxy steht, oder

$R^1$ und $R^2$ gemeinsam für einen Alkandioxy-Rest stehen,

X für Wasserstoff oder Chlor steht,

$R^3$ für die Gruppierung 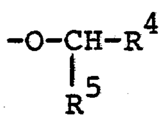 steht,

in welcher

$R^4$ für Wasserstoff oder für gegebenenfalls substituiertes Alkyl steht und

$R^5$ für einen gegebenenfalls substituierten Alkoxycarbonyl-Rest oder für einen gegebenenfalls substituierten Pyrazolrest steht; oder

$R^3$ für den Fall, daß X für Chlor steht, auch für gegebenenfalls substituierte Reste aus der Reihe Alkyl, Cycloalkyl, Alkylamino oder Cycloalkylamino steht.

2) Chlorierte Phosphorylmethylcarbonyl-Derivate der Formel (I) gemäß Anspruch 1, dadurch gekennzeichnet, daß

$R^1$ für gegebenenfalls einfach oder mehrfach durch Halogen oder $C_1$-$C_4$-Alkoxy substituierte Reste aus der Reihe Alkyl mit bis zu 6 Kohlenstoffatomen, Alkoxy mit bis zu 6 Kohlenstoffatomen, Aralkyl oder Aralkoxy mit 6 bis 10 Kohlenstoffatomen im Arylteil und bis zu 2 Kohlenstoffatomen im Alkylteil oder für gegebenenfalls einfach oder mehrfach durch Halogen, Nitro, $C_1$-$C_4$-Alkyl, $C_1$-$C_4$-Alkoxy und/oder $C_1$-$C_4$-Alkoxycarbonyl substituiertes Phenyl steht,

$R^2$ für gegebenenfalls einfach oder mehrfach durch Halogen oder $C_1$-$C_4$-Alkoxy substituiertes Alkoxy mit bis zu 6 Kohlenstoffatomen und Aralkoxy mit 6 bis 10 Kohlenstoffatomen im Arylteil und bis zu 2 Kohlenstoffatomen im Alkylteil steht, oder

$R^1$ und $R^2$ gemeinsam für einen verzweigten oder unverzweigten Alkandioxy-Rest mit 2 bis 5 Kohlenstoffatomen im Alkylteil stehen,

X für Wasserstoff oder Chlor steht,

$R^3$ für die Gruppierung $-\underset{\substack{|\\ R^5}}{OCH}-R^4$ steht,

in welcher

$R^4$ für Wasserstoff oder Alkyl mit bis zu 4 Kohlenstoffatomen steht und

$R^5$ für einen Alkoxycarbonyl-Rest mit bis zu 4 Kohlenstoffatomen im Alkylteil oder für einen gegebenenfalls substituierten Pyrazolyl-Rest

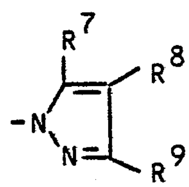

steht, wobei

$R^7$ und $R^9$ gleich oder verschieden sind und für Wasserstoff oder $C_1$-$C_4$-Alkyl stehen und

$R^8$ für Wasserstoff, Halogen oder $C_1$-$C_4$-Alkyl steht; oder

$R^3$ für den Fall, daß X für Chlor steht, auch für gegebenenfalls einfach oder mehrfach durch Halogen, $C_1$-$C_4$-Alkoxy und/oder $C_3$-$C_6$-Cycloalkyl substituiertes Alkyl mit bis zu 8 Kohlenstoffatomen oder für gegebenenfalls einfach

oder mehrfach durch $C_1$-$C_3$-Alkyl substituiertes Cycloalkyl mit 3 bis 6 Kohlenstoffatomen, Alkylamino mit bis zu 6 Kohlenstoffatomen im Alkylteil und Cycloalkylamino mit 3 bis 6 Kohlenstoffatomen steht.

3) Chlorierte Phosphorylmethylcarbonyl-Derivate der allgemeinen Formel (I) gemäß Anspruch 1, dadurch gekennzeichnet, daß

(A) $R^1$ für n-Propoxy, i-Propoxy, n-Butoxy, i-Butoxy, sec.-Butoxy oder tert.-Butoxy steht,

$R^2$ für n-Propoxy, i-Propoxy, n-Butoxy, i-Butoxy, sec.-Butoxy oder tert.-Butoxy steht,

X für Chlor steht und

$R^3$ für n-Propyl, i-Propyl, n-Butyl, i-Butyl, sec.-Butyl, tert.-Butyl, Cyclopropyl, n-Propylamino, i-Propylamino, n-Butylamino, i-Butylamino, sec.-Butylamino oder tert.-Butylamino steht,

oder

(B) $R^1$ für n-Propoxy, i-Propoxy, n-Butoxy, i-Butoxy, sec.-Butoxy, tert.-Butoxy, oder Phenyl steht,

$R^2$ für n-Propoxy, i-Propoxy, n-Butoxy, i-Butoxy, sec.-Butoxy oder tert.-Butoxy steht,

X für Chlor steht und

$R^3$ für die Gruppierung $-OCH\genfrac{}{}{0pt}{}{R^4}{R^5}$ steht,

in welcher

$R^4$ für Methyl oder Ethyl steht und

$R^5$ für Methoxycarbonyl, Ethoxycarbonyl, n-Propoxycarbonyl, i-Propoxycarbonyl, n-Butoxycarbonyl, i-Butoxycarbonyl, sec.-Butoxycarbonyl oder tert.-Butoxycarbonyl steht,

oder

(C) $R^1$ für n-Propoxy, i-Propoxy, n-Butoxy, i-Butoxy, sec.-Butoxy, tert.-Butoxy oder Phenyl steht,

$R^2$ für n-Propoxy, i-Propoxy, n-Butoxy, i-Butoxy, sec.-Butoxy oder tert.-Butoxy steht,

X für Wasserstoff oder Chlor steht und

$R^3$ für die Gruppierung $-OCH\genfrac{}{}{0pt}{}{R^4}{R^5}$ steht,

in welcher

$R^4$ für Wasserstoff, Methyl, Ethyl,

n-Propyl oder i-Propyl steht und

$R^5$ für einen gegebenenfalls substituierten Pyrazolyl-Rest

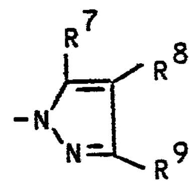

steht, wobei

$R^7$ und $R^9$ gleich oder verschieden sind und für Wasserstoff oder Methyl stehen und

$R^8$ für Wasserstoff oder Chlor steht.

4) Verfahren zur Herstellung von chlorierten Phosphorylmethylcarbonyl-Derivaten der Formel (I) gemäß Anspruch 1, dadurch gekennzeichnet, daß man

a) Dichlormethanphosphonsäure-Derivate der Formel (II),

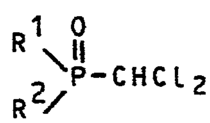

(II)

in welcher

$R^1$ und $R^2$ für gegebenenfalls substituierte

Reste aus der Reihe Alkoxy oder Aralkoxy stehen, oder

$R^1$ und $R^2$ gemeinsam für einen Alkandioxy-Rest stehen,

mit Säurehalogeniden der Formel (III),

$$R^3-\overset{O}{\overset{\|}{C}}-Hal \qquad (III)$$

in welcher

$R^3$ die oben angegebene Bedeutung hat und

Hal für Chlor oder Brom steht,

oder mit Isocyanaten der Formel (IV),

$$R^6-NCO \qquad (IV)$$

in welcher

$R^6$ für gegebenenfalls substituierte Reste aus der Reihe Alkyl oder Cycloalkyl steht,

gegebenenfalls in Gegenwart eines Säureakzeptors und gegebenenfalls in Gegenwart von Verdünnungsmitteln umsetzt, oder

b) Phosphorylmethylcarbonyl-Verbindungen der Formel (V),

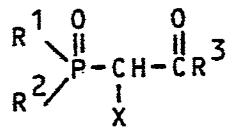

(V)

in welcher

$R^1$, $R^2$, $R^3$ und X die oben angegebenen Bedeutungen haben,

mit Chlorierungsmitteln gegebenenfalls in Gegenwart von Säureakzeptoren und gegebenenfalls in Gegenwart von Verdünnungsmitteln umsetzt.

5) Pflanzenschutzmittel, gekennzeichnet durch einen Gehalt an mindestens einem chlorierten Phosphorylmethylcarbonyl-Derivat der allgemeinen Formel (I) gemäß Anspruch 1.

6) Verfahren zur Herstellung eines Pflanzenschutzmittels, dadurch gekennzeichnet, daß man chlorierte Phosphorylmethylcarbonyl-Derivate der allgemeinen Formel (I) gemäß Anspruch 1 mit Streckmitteln und/oder oberflächenaktiven Mitteln vermischt.

Europäisches Patentamt

**EUROPÄISCHER RECHERCHENBERICHT**

0126249 Nummer der Anmeldung

EP 84 10 3626

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | EP-A-0 001 018 (E.I. DU PONT DE NEMOURS AND CO.) * Ansprüche * | 1-6 | C 07 F 9/40<br>C 07 F 9/32<br>C 07 F 9/65<br>A 01 N 57/18 |
| Y | EP-A-0 001 331 (E.I. DU PONT DE NEMOURS AND CO.) * Ansprüche * | 1-6 | |
| Y | CH-A- 310 406 (CIBA-GEIGY) * Insgesamt * | 1-6 | |
| Y | GB-A-1 197 883 (FARBENFABRIKEN BAYER AG) * Insgesamt * | 1-6 | |
| Y | GB-A- 744 360 (CIBA-GEIGY) * Insgesamt * | 1-6 | RECHERCHIERTE SACHGEBIETE (Int. Cl. 3) |
| Y | CH-A- 310 406 (CIBA-GEIGY) * Insgesamt * | 1-6 | C 07 F 9/00 |

-----

Der vorliegende Recherchenbericht wurde für alle Patentansprüche erstellt.

| Recherchenort | Abschlußdatum der Recherche | Prüfer |
|---|---|---|
| DEN HAAG | 24-07-1984 | BESLIER L.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82